# DEMANDE DE BREVET EUROPEEN

(11) **EP 0 562 146 A1**
(43) Date de publication de la demande: **29.09.1993**
(21) Numéro de dépôt: 92105282.5
(22) Date de dépôt: 27.03.1992
(51) Int. Cl.: G01N 33/24, G01V 5/02

(54) **Procédé et appareil de diagraphie d'un sondage géologique**

(71) Demandeur: DIASOL HOLDING S.A., L-8472 Eischen (LU)
(72) Inventeur: Naa, Paul, L-8472 Eischen (LU)
(74) Mandataire: Meyers, Ernest

(57) **Abrégé**

L'analyse des échantillons délivrés sous forme de carottes par un appareil carottier dans un puits de forage sont effectuées à l'aide d'un appareil de diagraphie mobile que l'on déplace le long des carottes précédemment prélevées dans le puits et couchées à la surface du sol sur le site de forage. Les mesures sont enregistrées en fonction de la position et du déplacement de l'appareil de diagraphie le long des carottes.

## Description

La présente invention concerne un procédé de diagraphie d'un sondage géologique, selon lequel on analyse des échantillons délivrés sous forme de carottes prélevées par un appareil carottier dans un puits de forage. L'invention concerne également un appareil pour la mise en oeuvre de ce procédé.

L'invention vise essentiellement la prospection pétrolière et minière pour déterminer, par des méthodes scientifiques fiables, l'emplacement de nouvelles réserves ou gisements ou l'étendue de gisements déjà exploités en vue de leurs extensions.

Parmi les nombreuses méthodes de forage, l'invention vise plus particulièrement le procédé mettant en oeuvre le carottage. Ce procédé consiste à prélever, au cours du forage, des échantillons ou carottes à l'aide d'un appareil carottier dans le fond du puits à des profondeurs supposées intéressantes en vue d'analyses plus détaillées. Ces carottes dont les longueurs peuvent varier entre 5 et 18m doivent être remontées à la surface après chaque prélèvement. Elles sont ensuite sectionnées sur le site de forage avec leur gaine de protection tube de carottage et les sections qui méritent une analyse plus approfondie sont ensuite envoyées dans un laboratoire avec des instruments d'analyses perfectionnés. Ces laboratoires se trouvent, en général, à de grandes distances du forage et nécessitent des transports aériens pour acheminer les échantillons. Ceci constitue un grave handicap, étant donné que non seulement le transport en soi des échantillons est onéreux, mais, surtout, le forage ne peut être utilement poursuivi qu'après retour des résultats des analyses. Or, compte tenu du matériel mis en oeuvre, une interruption de forage est extrêmement coûteuse.

Par ailleurs, ce système de diagraphie par carottage et analyse en laboratoire ne peut être appliqué seul, car il doit être complété par des analyses "d'appoint" permettant, d'abord de déterminer les endroits intéressants pour effectuer un carottage dans le puits et, ensuite, pour déterminer les sections des carottes qui seront envoyées en laboratoire à des fins d'analyses plus approfondies. A cet effet, il est connu d'utiliser un dispositif tel que proposé par le document EP-A-0 043 313 selon lequel on détecte le rayonnement gamma provenant de la radioactivité naturelle ou provoqué de formations rocheuses d'un puits de forage. Un outil de diagraphie par rayonnement gamma suspendu à un câble est descendu au fond du puits et explore lors de sa remontée les différentes formations rocheuses. Ce détecteur comprend, de manière connue en soi, un scintillateur, un photomultiplicateur, un amplificateur linéaire et un analyseur de hauteur d'impulsions divisé en diverses fenêtres aux moyens de comparateurs ayant des seuils prédéterminés permettant de calculer les teneurs en thorium, uranium et potassium de ces formations rocheuses. Le rayonnement gamma naturel produit dans le scintillateur des photons qui sont détectés dans le photomultiplicateur et amplifiés dans l'amplificateur linéaire pour produire les impulsions de mesure. Etant donné que les formations géologiques qui sont ainsi traversées par l'outil de diagraphie sont celles dans laquelle a été prélevée précédemment une carotte, on se base sur cette diagraphie pour l'échantillonnage des carottes, c'est-à-dire pour choisir les sections les plus intéressantes qui seront expédiées en laboratoire pour analyses approfondies.

Ce système de diagraphie présente toutefois plusieurs inconvénients. D'abord la diagraphie doit être effectuée en absence d'appareil carottier dans le puits de sondage. Il faut donc retirer préalablement l'appareil carottier du puits et interrompre le prélèvement des carottes avant de descendre l'outil de diagraphie jusqu'au fond du puits de forage. Si l'on tient compte du fait que les puits de forage peuvent avoir une profondeur jusqu'à 5000 m et même au-delà, on comprend que ce genre de diagraphie occasionne une grande perte de temps, d'autant plus qu'une opération de forage et de carottage, interrompue par une diagraphie, ne peut être poursuivie qu'après la remontée et le retrait de l'outil de diagraphie.

En outre, les résultats des mesures effectuées par une telle diagraphie ne sont pas toujours fiables, notamment à cause du fait que l'exploration des couches géologiques par l'outil de diagraphie ne peut pas être observée visuellement. Il est, par exemple, possible que les parois du puits soient recouvertes par de la boue de forage, ce qui peut fausser les mesures. Il est également possible que la carotte, une fois à la surface, ne corresponde plus physiquement aux couches géologiques qu'elle est sensée représenter. Par suite de l'érosion dans le tube carottier, la carotte peut, par exemple, devenir plus courte que l'épaisseur des couches géologiques sur lesquelles elle a été prélevée. Si, par exemple, le forage traverse un vide, celui-ci disparaîtra dans le tube carottier, de sorte que les différentes sections de la carotte seront décalées par rapport aux emplacements des couches géologiques correspondantes. Autrement dit, lors de l'échantillonnage des carottes pour sélectionner les tronçons à expédier au laboratoire, on n'est pas toujours certain de choisir réellement les tronçons révélés intéressants par la diagraphie au fond du puits, d'autant plus que l'on ne peut pas observer les carottes, vu quelles sont découpées avec le tube carottier protecteur.

Le but de la présente invention est de proposer un nouveau procédé et appareil de diagraphie qui permettent d'éliminer ou, du moins de réduire sensiblement les pertes de temps et de fournir des résultats de mesure plus fiables que les procédés classiques.

Pour atteindre cet objectif, le procédé proposé par la présente invention est caractérisé en ce que l'on utilise un appareil de diagraphie mobile que l'on déplace le long des carottes précédemment prélevées dans le puits et couchées à la surface du sol sur le site de forage et en ce que l'on enregistre les mesures en fonction de la position et du déplacement de l'appareil de diagraphie le long des carottes. Au lieu que ce soit l'appareil de mesure qui descende observer les terrains en place, ce sont les échantillons de terrain (ou carottes) qui remontent près de l'appareil de mesure.

Les mesures effectuées sont essentiellement des mesures basées sur la radioactivité naturelle des carottes, mais ces mesures, sont avantageusement complétées par des mesures de radioactivité provoquée par une source de rayonnement gamma et des mesures électromagnétiques de la résistivité.

Alors que la mesure basée sur le rayonnement gamma naturel fournit des renseignements relatives aux teneurs des formations en argile, les mesures basées sur une radioactivité provoquée et les mesures électro-magnétiques fournissent des informations stratigraphiques et pétrographiques beaucoup plus complètes, notamment en ce qui concerne la densité et la porosité des formations, respectivement la résistivité et la conductivité qui renseignent sur les caractéristiques des fluides contenus dans les formations rocheuses.

Toutefois, l'avantage prépondérant est que ces informations sont obtenues directement sur le site du forage, c'est-à-dire beaucoup plus rapidement que si les échantillons sont envoyés dans un laboratoire pour analyses approfondies, même si ces analyses approfondies ne sont pas exclues par le procédé proposé. Au contraire, étant donné que la diagraphie est effectuée directement sur la carotte, il est possible de déterminer avec beaucoup plus d'exactitude les sections intéressantes de celles-ci, qui seront sélectionnées pour analyses en laboratoire. En outre, les analyses effectuées sur place par le procédé de diagraphie proposé par la présente invention permettent de fournir des renseignements suffisamment utiles pour la poursuite, soit du forage destructif, soit de carottage supplémentaire.

La possibilité de corrélation de la colonne lithologique du puits avec les informations relatives à des puits avoisinants ou avec des résultats attendus sont extrêmement utiles pour le choix de la profondeur des carottages suivants, notamment en vue des mesures de porosité, de perméabilité et de teneur en argile.

L'invention propose également un appareil de diagraphie pour la mise en oeuvre de ce procédé, qui est caractérisé en ce qu'il est monté sur un chariot mobile pourvu de galets dont au moins un est associé à un encodeur optique fournissant des impulsions en fonction du déplacement du chariot qui est conçu pour pouvoir évoluer au-dessus d'une carotte couchée par terre et comprenant un compartiment inférieur renfermant au moins un détecteur de rayonnement gamma et un compartiment supérieur renfermant l'alimentation électrique et les commandes et une mémoire pour enregistrer les mesures fournies par le détecteur en fonction des impulsions fournies par l'encodeur du déplacement du chariot.

Le détecteur peut être constitué par un scintillateur et un photomultiplicateur disposés dans le compartiment inférieur et noyés dans une mousse ou résine adaptée.

Les parois latérales et supérieures du compartiment inférieur comportent, de préférence, une couche de plomb formant écran d'absorption pour les rayonnements gamma parasites.

L'alimentation électrique est une alimentation autonome par batteries rechargeables.

Pour effectuer des mesures de radioactivité provoquée, le compartiment inférieur peut comporter une cartouche amovible avec une source de rayonnements gamma.

Pour effectuer les mesures de résistivité, il est possible d'engendrer un champ magnétique à l'aide d'une bobine disposée dans le compartiment inférieur.

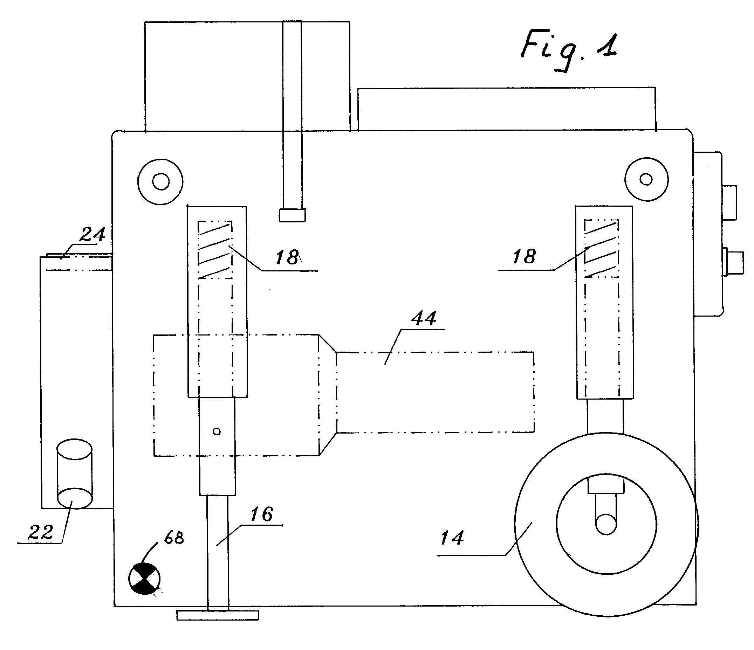

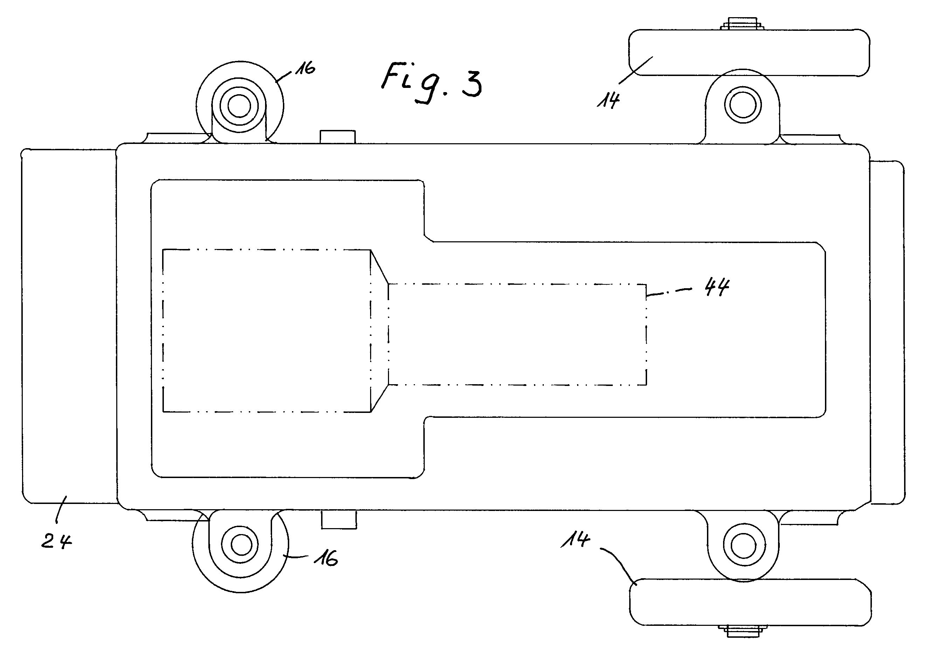

D'autres particularités et caractéristiques de l'invention ressortiront de la description détaillée d'un mode de réalisation préféré, présenté ci-dessous, à titre d'illustration, en référence aux dessins annexés dans lesquels:
- la figure 1 montre une vue de profil d'un appareil de diagraphie mobile selon la présente invention;
- la figure 2 montre schématiquement une coupe verticale à travers l'appareil de diagraphie;
- la figure 3 montre schématiquement une vue en plan de l'appareil;
- la figure 4 montre un schéma synoptique des parties essentielles de l'appareil de diagraphie et
L'appareil de diagraphie représenté sur les figures 1 à 3 est monté sur un chariot mobile porté par deux galets 14, par exemple en matière synthétique, et par deux béquilles 16 servant d'appui lorsque l'appareil n'est pas en opération. Les galets 14 sont, de préférence, réglables en hauteur. La suspension de l'appareil sur les galets 14 et les béquilles 16 est réalisée par des amortisseurs 18.

L'appareil, dans sa version expérimentale, n'a qu'un poids de l'ordre de 15 kilos et peut être muni d'une anse non représentée pour faciliter son transport.

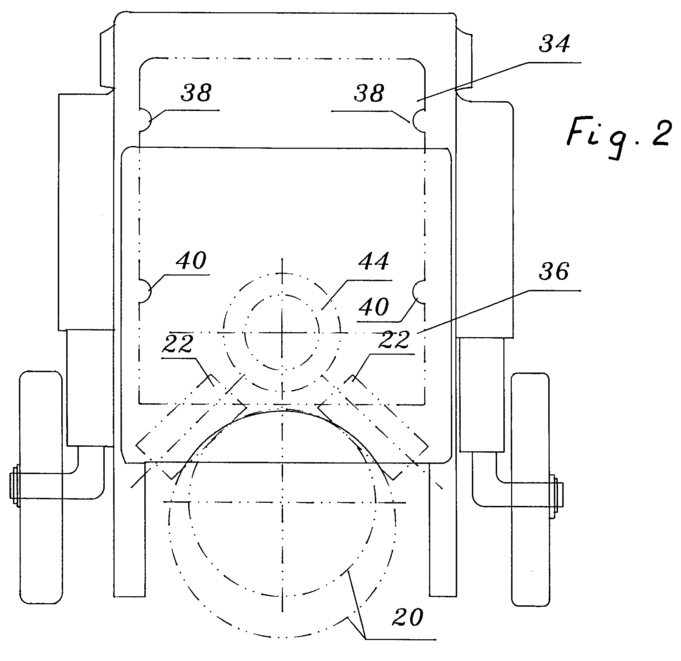

En opération l'appareil est poussé manuellement au-dessus d'une carotte 20 ce qui n'exclut toutefois pas la possibilité de prévoir un chariot autotracté. Grâce au fait que les galets 14 sont réglables en hauteur il est possible de sonder des carottes 20 de diamètres différents (voir figure 2).

Lorsque l'appareil est poussé au-dessus d'une carotte 20 pour effectuer un sondage, les béquilles 16 sont retractées dans leurs supports respectifs et y maintenues p.ex. simplement à l'aide d'une goupille. Le support de l'appareil du côté des béquilles est alors assuré par deux galets de roulement inclinés 22 prenant directement appui sur la partie supérieure de la carotte 20.

Pendant le sondage il est nécessaire d'effectuer les mesures en fonction de la position de l'appareil afin de pouvoir faire la corrélation entre les mesures et la profondeur des couches géologiques dans lesquelles la carotte a été prélevée. A cet effet l'appareil est pourvu d'un encodeur de profondeur, connu en soi, par exemple électro-optique qui fournit des impulsions représentatives du déplacement de l'appareil. Cet encodeur représenté par 56 sur la figure synoptique 4 se trouve dans un compartiment 24 à l'avant de l'appareil et est actionné directement par l'un des galets 22. Les impulsions produites par cet encodeur lors du déplacement de l'appareil 10 au-dessus d'une carotte sont comptées par l'électronique de l'appareil. Le nombre de ces impulsions fournit une indication quant à l'avancement et à la position de l'appareil. Dans un appareil expérimental, l'encodeur a une résolution de 1 mm, quoique les mesures ne soient effectuées que tous les 10 mm.

Les parties essentielles de l'appareil sont logées dans un tiroir supérieur 34 et dans un tiroir inférieur 36. Ces deux tiroirs 34 et 36 sont suspendus dans des glissières latérales 38 respectivement 40 ce qui leur permet d'être dégagés facilement, le tiroir 36 avec le compartiment 24 de l'encodeur vers l'avant et le compartiment supérieur 34 vers l'arrière.

Le tiroir supérieur 34 contient les composants électroniques essentiels. Pour préserver ces composants des influences atmosphériques, ce compartiment supérieur 34 peut contenir un chauffage de protection anti-condensation.

Le tiroir inférieur 36 contient un détecteur 44 de la radioactivité gamma de la carotte 20. A cause de la fragilité de ce détecteur 44 et de ses composants, celui-ci est de préférence noyé dans une mousse remplissant tout le tiroir 36 afin d'absorber les chocs éventuels et de réduire les risques d'endommagement du détecteur. Les parois latérales et supérieures du tiroir 36 peuvent être pourvues, du moins à certains endroits, d'une couche de plomb formant écran aux rayonnements gamma extérieurs qui pourraient fausser ou masquer les mesures de la radioactivité naturelle de la carotte 20.

Pour assurer un fonctionnement autonome de l'appareil l'alimentation électrique est assurée par des batteries. Celles-ci peuvent être logées dans la partie supérieure de l'appareil, au-dessus du tiroir 34. L'appareil peut également comporter une prise pour le rechargement de l'appareil.

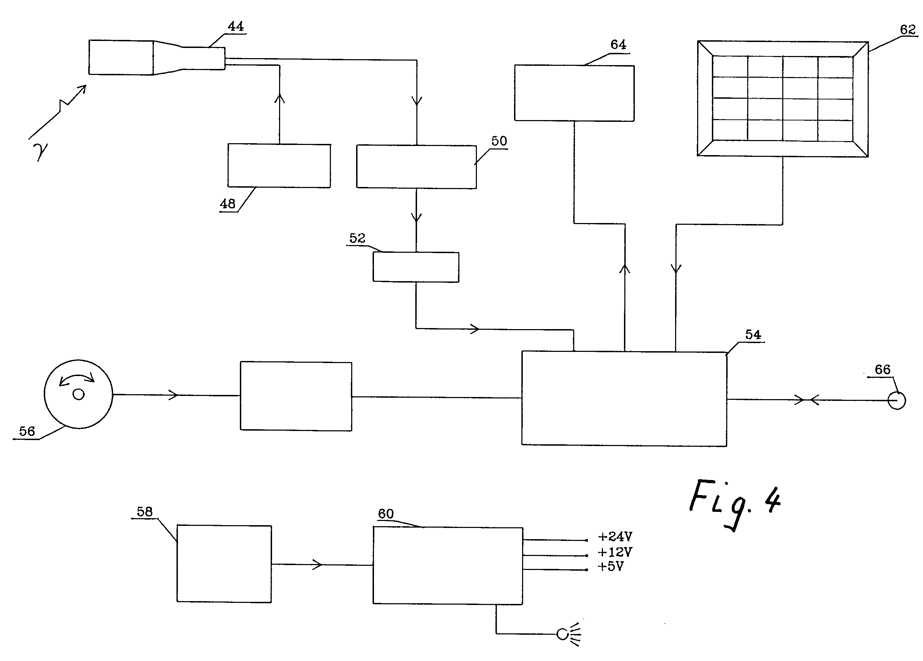

Le détecteur 44 peut être un détecteur connu en soi du genre de ceux utilisés dans les outils de diagraphie qui sont descendus au fond du puits. Un tel détecteur comporte un scintillateur constitué d'un cristal d'iodure de sodium relié à un tube photomultiplicateur. Le fonctionnement est résumé par le schéma synoptique de la figure 4 montrant les composants électroniques dans le compartiment 34. Le scintillateur 44, réagit à la radiation gamma par l'émission de photons qui sont détectés et multipliés dans le photo-multiplicateur alimenté par une source de haute tension 48. Ce sont ces particules de photons ou de lumière qui sont détectées, sélectionnées et comptées à l'aide d'un ou de plusieurs discriminateurs 50 et d'un ou de plusieurs compteurs 52 et ensuite mises en mémoire dans une unité centrale 54 à microprocesseur sous forme de nombres par secondes et en fonction des données fournies par l'encodeur 56 et représentatives de la position de l'appareil, c'est-à-dire de la profondeur des couches explorées. La mémoire du microprocesseur 54 est une mémoire permanente d'au moins 64 K RAM capable de mémoriser les données correspondant à une distance de au moins 80m et échantillonnées à intervalles de 1cm. Le dépouillement de ces données fournit des renseignements au sujet de la composition des carottes, notamment en argile, en schiste, ou en tout autre élément radio-actif.

Deux batteries 58 de 12 volts chacune alimentent un circuit de distribution 60 pour fournir le courant d'alimentation des divers composants électriques et de la source de haute tension, qui amplifie les 24 volts en 1000 volts DC nécessaires au fonctionnement du photomultiplicateur.

L'opérateur peut communiquer avec l'appareil à l'aide d'un clavier de commande 62 et d'un moniteur 64 constitués d'un écran LCD, prévus, tous les deux, dans ou sur le couvercle de l'appareil. Le clavier 62 peut comporter 16 touches de commande, à savoir 10 touches alphanumériques, 2 touches "on-off", une touche "delete" et 3 touches de fonction "set", "test" et "record".

La touche de fonction "set" permet à l'opérateur de calibrer l'appareil sur la profondeur initiale, de choisir la cadence d'échantillonnage et de contrôler l'état de remplissage de la mémoire du microprocesseur afin de vérifier la capacité de la mémoire disponible pour effectuer une diagraphie.

Avec la fonction "test" l'opérateur peut tester le détecteur sans enregistrer les données et sans déplacer l'appareil. Ceci permet de contrôler le fonctionnement correct de l'appareil et de vérifier si les impulsions parasites provenant de la radioactivité naturelle ambiante ne sont pas trop importantes.

La fonction "record" permet à l'opérateur de déclencher le processus de diagraphie d'une carotte. Pendant que l'appareil est déplacé au-dessus d'une carotte, les mesures de radiation sont enregistrées à la cadence d'échantillonnage sélectionnée auparavant et mémorisées en fonction des valeurs de profondeur fournies par l'encodeur. Une marque apparaît sur l'écran LCD 64 chaque fois qu'un échantillon de mesure est enregistré. La vitesse de diagraphie est, de préférence, réglée de manière qu'un enregistrement ait lieu par exemple à la cadence de une seconde, ou tous les 10mm.

L'appareil de diagraphie est prévu de manière à pouvoir être raccordé à un ordinateur PC portable indépendant 66. A la fin d'une diagraphie, ou lorsque la mémoire du système 54 est pleine, les données enregistrées sont transférées dans le PC 66. Le logiciel utilisé pour effectuer cette opération de transfert est un programme spécial développé sous "WINDOWS" (marque déposée). Lors du transfert des données dans le PC, celles-ci apparaîtront automatiquement sur le moniteur de celui-ci sous forme d'une ou de plusieurs courbes sur l'abcisse desquelles figurent les valeurs des rayonnements gamma et sur l'ordonnée de laquelle figure la profondeur.

Le logiciel permet de modifier, au gré de l'opérateur, l'échelle de l'abscisse et de l'ordonnée. De même, il est possible de modifier le nombre des lignes de grille horizontales et verticales ainsi que la couleur de l'affichage. Il est également possible de prévoir un filtre pour l'affichage en ordonnée de manière que la courbe ne soit pas trop nivelée ou trop hérissée.

Les résultats de la diagraphie peuvent être mémorisés sur la mémoire principale de l'ordinateur PC ou être sortis sur papier par une imprimante. A cette occasion, il est possible d'utiliser du papier permettant l'impression automatique d'autres données concernant, par exemple, l'emplacement du puits, la profondeur de carottage, le nom du maître d'oeuvre, etc.

Il est également possible de combiner les résultats de diagraphies différentes afin d'obtenir une courbe continue d'exploration de plusieurs carottes.

L'appareil peut également comporter des moyens de mesure de radioactivité provoquée dans la carotte pour compléter les mesures de la radioactivité naturelle et fournir des mesures complémentaires au sujet de la carotte. A cet effet, on peut prévoir une source de radioactivité au Caesium contenue dans une cartouche 68 logée dans la paroi de l'appareil.

Les mesures électromagnétiques induites de la résistivité peuvent être effectuées de manière connue en soi à l'aide d'une bobine placée dans le compartiment inférieur 36.

## Revendications

1. Procédé de diagraphie d'un sondage géologique, selon lequel on analyse les échantillons délivrés sous forme de carottes par un appareil carottier dans un puits de forage, caractérisé en ce que l'on utilise un appareil de diagraphie mobile que l'on déplace le long des carottes précédemment prélevées dans le puits et couchées à la surface du sol sur le site de forage et en ce que l'on enregistre les mesures en fonction de la position et du déplacement de l'appareil de diagraphie le long des carottes.

2. Procédé selon la revendication 1, caractérisé en ce que l'on mesure la radioactivité naturelle des carottes.

3. Procédé selon la revendication 1, caractérisé en ce que l'on détermine la densité par une mesure de la radioactivité provoquée par une source de rayonnement gamma dans la carotte.

4. Procédé selon la revendication 1, caractérisé en ce que l'on détermine la résistivité par des mesures électromagnétiques.

5. Appareil de diagraphie pour la mise en oeuvre du procédé selon l'une quelconque des revendications 1 à 4, caractérisé en ce qu'il est monté sur un chariot mobile pourvu de galets (14, 22) dont au moins un est associé à un encodeur fournissant des impulsions en fonction du déplacement du chariot qui est conçu pour pouvoir évoluer au-dessus d'une carotte (20) couchée par terre et comprenant un compartiment inférieur (36) renfermant au moins un détecteur (44) de rayonnement gamma et un compartiment supérieur (34) renfermant l'alimentation électrique et les commandes, ainsi qu'une mémoire pour enregistrer les mesures fournies par le détecteur (44) en fonction des impulsions fournies par l'encodeur (56) du déplacement du chariot (12).

6. Appareil selon la revendication 5, caractérisé en ce que le détecteur (44) est constitué par un scintillateur et un photomultiplicateur noyés dans une mousse.

7. Appareil selon la revendication 5, caractérisé en ce que les parois latérales et supérieure du compartiment inférieur (36) comportent une couche de plomb formant écran d'absorption des rayonnements gamma.

8. Appareil selon la revendication 5, caractérisé en ce que l'alimentation électrique est une alimentation autonome par batteries.

9. Appareil selon la revendication 5, caractérisé en ce que le compartiment supérieure renferme un chauffage de protection anti-condensation.

10. Appareil selon la revendication 5, caractérisé par des moyens d'induction et de mesure de champs électromagnétiques disposés dans le compartiment inférieur.

11. Appareil selon la revendication 5, caractérisé par une cartouche amovible avec un émetteur de rayonnements gamma pouvant être accrochée sur une paroi de l'appareil

12. Appareil selon la revendication 5, caractérisé en ce que les compartiments inférieurs et supérieurs sont constitués sous forme de tiroirs coulissants étanches.

13. Appareil selon la revendication 5, caractérisé en ce que deux galets (22) sont disposés pour rouler sur la carotte (20) et deux autres (14) évoluant sur le sol sont réglables en hauteur.
